**Europäisches Patentamt**

**European Patent Office**

**Office européen des brevets**

(19)

(11) Numéro de publication: **0 222 644**
**B1**

(12) **FASCICULE DE BREVET EUROPEEN**

(51) Int. Cl.⁵: **C12M 1/02**, C12M 3/00

(45) Date de publication du fascicule du brevet:
**14.11.90**

(21) Numéro de dépôt: **86402265.2**

(22) Date de dépôt: **10.10.86**

(54) **Procédé de culture "in vitro".**

(30) Priorité: **11.10.85 FR 8515289**

(43) Date de publication de la demande:
**20.05.87 Bulletin 87/21**

(45) Mention de la délivrance du brevet:
**14.11.90 Bulletin 90/46**

(84) Etats contractants désignés:
**DE GB**

(56) Documents cités:
**DE-A- 2 300 567**
**FR-A- 1 598 245**
**GB-A- 2 089 837**
**US-A- 4 442 206**

**Phytopathology(1974), pp 903-905, vol. 64**

(73) Titulaire: **UNIVERSITE DE NANTES, 1 Quai de Tourville B.P. 1026, F-44035 Nantes Cedex(FR)**

(72) Inventeur: **Duroselle, Patrick Marie Bruno Pierre, 4 Avenue du Parnasse, F-44800 St-Herblain(FR)**

(74) Mandataire: **Phélip, Bruno et al, c/o Cabinet Harlé & Phélip 21, rue de La Rochefoucauld, F-75009 Paris(FR)**

## Description

L'invention est relative à un procédé de culture "in vitro", en particulier, mais pas exclusivement de cellules homéothermes, minimisant les interventions manuelles et l'instrumentation lourde habituelle à cette technique, notamment lors des observations microscopiques, des analyses chimiques du milieu, des passages de la culture développée dans de plus grandes enceintes.

L'invention concerne également une installation pour la mise en oeuvre de ce procédé.

Bien que principalement destinée aux cellules d'organismes homéothermes, ses applications peuvent s'étendre à toutes les cultures tissulaires ou cellulaires animales ou végétales y compris en particulier aux cultures bactériologiques notamment celles des flores anaérobies.

Les techniques actuelles de culture "in vitro", en particulier de cellules provenant d'organismes homéothermes, nécessitent une étuve et l'observation de la culture ne peut se faire qu'en dehors de l'étuve, au prix de manipulations multiples, délicates et dispendieuses augmentant en particulier leur coût de mise en oeuvre et les risques de pollution bactériologique ou virale.

Des installations ont été développées pour réaliser une observation en continu de la culture ; mais il s'agit là d'un matériel spécifique, lourd et surtout très onéreux constitué notamment d'une étuve bâtie autour des moyens d'observation.

Des travaux ont été menés pour établir une culture dans une chambre de petite dimension qui est conformée pour permettre l'observation simultanée. De telles chambres sont, par exemple, décrites dans l'ouvrage de John Paul, "CELL and TISSUE CULTURE" 5ème édition, p. 341-343 et notamment dans la revue "PHYTOPATHOLOGY" 64, 903-905 où il est question d'une chambre perfusable perfectionnée.

Cette dernière, du type SYKES-MOORE, est constituée d'un boitier cylindrique plat, en deux parties, qui renferme et bride deux parois circulaires en verre séparées par un joint annulaire épais. La culture est établie entre les plaques de verre et alimentée par perfusion à travers le boitier et le joint en silicone. Ces chambres permettent le développement de cultures en condition de perfusion et l'observation microscopique et microcinématographique simultanée mais cette observation ne peut pas se faire en continu pendant de longues périodes, hors d'une étuve.

On a donc tenté d'améliorer la durée d'observation de la culture se développant dans une telle chambre au moyen d'un dispositif de thermo-régulation de la température de la chambre. Ce dispositif, adapté à la chambre SYKES-MOORE, consiste principalement en une enveloppe annulaire disposée autour du boitier cylindrique, et dans laquelle circule notamment un fluide caloporteur.

Ce dispositif présente cependant l'inconvénient d'empêcher la perfusion et la thermostation simultanée car il masque les orifices d'introduction des seringues.

Par ailleurs, ce dispositif ne permet pas d'assurer une thermostation suffisante pour réaliser une culture. Cette dernière se développe en effet le plus souvent sur la plaque du fond de la chambre ou du pot de culture ; or cette plaque subit des variations de température selon l'ambiance et l'environnement immédiat.

L'invention a pour but de pallier à ces inconvénients et notamment de permettre une croissance cellulaire dans des enceintes closes autorisant son observation et sa photographie continue, en particulier sur un microscope inverse usuel, sans infliger aux cellules des risques de pollution et des variations d'environnement, principalement thermiques, néfastes à leur croissance et à leur multiplication.

Un autre but de l'invention est de fournir une installation pour la mise en oeuvre du procédé, supprimant la nécessité d'appareillage lourd et donc particulièrement adapté à la culture de petites ou moyennes quantités cellulaires, tissulaires ou bactériennes telles que celles manipulées en particulier dans la recherche, notamment odontologique.

Un autre but de l'invention est de fournir une installation adaptée aussi bien au laboratoire de recherche qu'à l'industrie pour assurer leurs besoins en particulier en ce qui concerne par exemple le suivi de la qualité d'un biomatériau quant à sa cytotoxicité.

L'invention se propose en complément, mais non exclusivement, de résoudre les problèmes spécifiques concernant en particulier la constitution de "banques" de lignées primaires de cellules, la survie et l'observation de cellules post-mitotiques, la culture, l'observation et l'isolement de prélèvements bactériologiques en particulier ceux comportant une flore anaérobie plus ou moins stricte sans recourir à la lourde installation usuelle.

L'invention concerne à cet effet un procédé de culture "in vitro ", en particulier de cellules homéothermes, consistant à établir la culture dans un pot approprié comportant un milieu nutritif et muni de moyens de thermostation, en vue de l'observation de l'évolution de la culture. Ce procédé est caractérisé en ce qu'il consiste à établir, au moins sous le pot de culture, une circulation de fluide caloporteur que l'on maintient à température adaptée à l'environnement et à l'expérimentation ; ce fluide caloporteur est transparent tout comme les parois du pot de culture, principalement les parois horizontales, ce qui permet l'observation continue de l'évolution de la culture, en-dehors de toute étuve.

Le pot de culture peut avantageusement être disposé dans une enceinte thermostatée indépendante permettant une circulation de fluide caloporteur autour et sous ledit pot. Ce dernier peut également être alimenté par une circulation de fluides nutritifs nécessaires à la culture.

L'invention se propose également de fournir une installation pour la mise en oeuvre d'un procédé de culture "in vitro" dans des conditions particulières de microgravité ; c'est-à-dire pour des expérimentations dans l'espace au sein d'une navette ou station orbitale par exemple.

L'invention concerne le fait d'établir une circulation de fluide caloporteur pressurisé dans la chambre de thermostation, permettant en particulier, le développement de la culture dans des conditions de micro-gravité.

L'invention concerne également une installation mettant en oeuvre le procédé pour réaliser notamment une observation de la culture. Cette installation est constituée d'au moins un pot de culture sous le fond duquel circule une lame de fluide caloporteur transparent provenant de et retournant à une unité centrale de thermostation. Cette lame parcourt un espace creux prévu entre le fond du pot et le fond d'une chambre de thermostation ; au moins les faces horizontales de la chambre thermostatée et du pot de culture sont réalisées en matière transparente, de manière à permettre l'observation, en particulier optique, de la culture.

De façon avantageuse, le pot de culture est muni de moyens d'alimentation continue en fluides nutritifs, et, est associé à une chambre thermostatée définissant de toute part, sauf éventuellement à leur face supérieure, un espace destiné à la circulation d'un fluide caloporteur provenant de et retournant à une unité centrale de thermostation.

L'invention concerne encore une installation dont le ou les pots de culture et la chambre de thermostation sont pourvus d'orifices apairés susceptibles d'être mis en communication avec les réservoirs de fluide nécessaire au déroulement de la culture, notamment à l'aide d'un cathéter.

L'invention concerne également la circulation du milieu de culture entre un réservoir oxygénateur et le pot de culture ; cette circulation est assurée, pour certaines applications telle que la perfusion de la culture en circuit ouvert, à partir d'une réserve de gaz sous pression servant à la fois à l'oxygénation du milieu et à sa propulsion au travers du pot de culture en fonction des nécessités de celle-ci.

L'ensemble de l'installation proposée et de son procédé de mise en oeuvre offre en particulier, la possibilité d'affranchir la culture de la nécessité d'une étuve à atmosphère contrôlée tout en permettant son observation continue ou discontinue et donc éventuellement l'automatisation de son pilotage sans immobiliser pour cette observation un appareil d'observation comme par exemple un microscope dans une enceinte thermostatée.

De plus le dispositif de perfusion ou de circumfusion évite les nombreuses manipulations sources de pollution bactérienne ou autre et l'augmentation du prix de revient.

Enfin, l'utilisation de chambres thermostatées homothétiques de dimension croissante et de culture sur lamelles de dimensions adéquates et pré-fragmentées permet de minimiser les interventions humaines au strict minimum lors du développement de la culture et d'éliminer les artéfacts d'origine biochimique comme les digestions enzymatiques modifiant les parois cellulaires lors des manipulations classiques de repiquage.

L'invention concerne encore l'installation comportant, autour du ou des pots de culture, un espace destiné à la circulation en circuit fermé et pressurisé, d'un fluide caloporteur transparent.

D'autres caractéristiques de l'invention seront encore mises en évidence au cours de la description qui suit, eu égard aux dessins annexés, bien entendu donnés à titre purement indicatif et de ce fait n'étant pas à l'échelle, ni côtés.

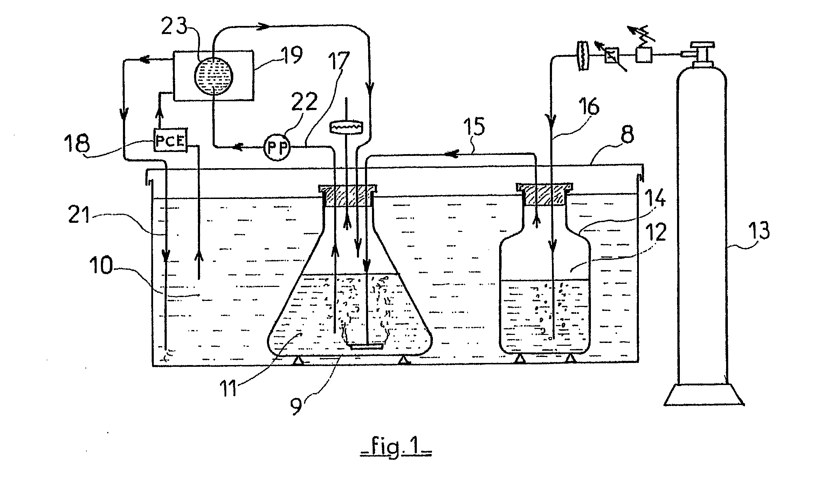

La figure 1 montre l'organisation générale du procédé dans le cas d'une culture à circumfusion avec oxygénation fournie par le milieu de culture.

La figure 2 montre l'organisation générale du procédé dans le cas d'une culture simplement perfusée, avec recueil du milieu de culture usagé et dans sa mise en oeuvre la plus simple sans pompe de perfusion, la seule pression du gaz d'oxygénation étant utilisée pour propulser le milieu au travers du pot de culture.

La figure 3 montre la partie centrale du dispositif périphérique de culture dit "pots de culture" dans une de ses réalisations possibles où le gazage de la culture se fait, non par dissolution dans le milieu au niveau du réservoir central, mais au niveau même de la culture comme c'est le cas dans les procédures classiques utilisant par exemple des flacons plats type CARREL.

La figure 4 représente en perspective un exemple de chambre thermostatée ouverte contenant deux pots de culture tels que précédemment décrits sur la figure 3.

Les figures 5 et 6 montrent en coupe transversale et longitudinale la même "chambre thermostatée" fermée et contenant deux "pots de culture" à perfusions gaz et milieu indépendantes ainsi que le dispositif de joint et de circulation du fluide caloporteur de thermostation.

La figure 7 montre une vue de dessus du même dispositif coupé au niveau des orifices de perfusion, la coupe A étant seule valable si le dispositif est utilisé avec un gazage du milieu dans l'unité centrale telle que décrite sur les figures 1 et 2, le pot de culture ne comportant qu'une seule paire d'orifices de cathétérisme.

Le procédé de culture consiste donc à utiliser une unité centrale formée d'un bain thermostaté (10) de laboratoire, de puissance adaptée, dont la fonction est d'une part de thermostater le milieu de culture (11) et le gaz nécessaire à cette culture (12) provenant d'une bouteille (13) de mélange approprié tel que air + $CO_2$. Cette unité centrale comporte de ce fait en son sein les réservoirs (9) et humidificateurs (14) nécessaires selon le procédé choisi et reliés entre eux de la façon convenable par des tubulures adaptées (15), (16), (17).

D'autre part l'unité centrale fournit aux unités périphériques de culture (19) un fluide caloporteur, tel que de l'eau, à température constante, propulsé par des pompes (18) de débit convenable, ce fluide caloporteur ayant pour objet de thermostater lesdites unités périphériques (19) quelle que soit leur situation, en particulier, sur la platine d'un microscrope à contraste de phase (non représenté), ou sur tout autre appareil d'observation, mais aussi sur la platine supérieure (8) de l'unité centrale.

Le captage du fluide de thermostation se fait de préférence en un point voisin du capteur de régulation de température du bain thermostatique (non représenté) et son retour est assuré par une tubulure appropriée (21).

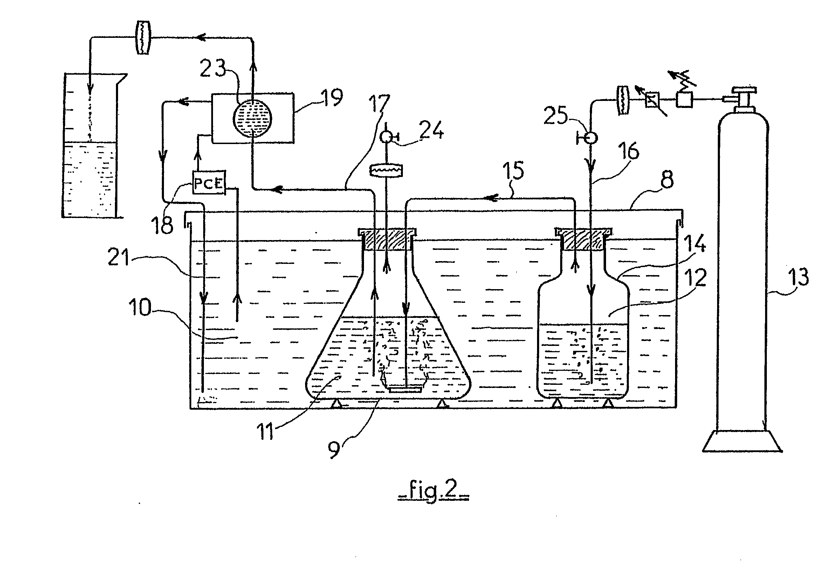

Des pompes péristaltiques (22), pousse-seringues (non représenté), ou comme sur la figure 2 la simple pression du gaz d'oxygénation sont utilisés pour la perfusion du milieu de culture (11) au travers des pots de culture (23) assurant ainsi aux cellules l'apport nutritif.

Cette perfusion peut se faire en circuit ouvert, avec recueil éventuel du milieu de culture et son utilisation, en particulier pour l'analyse (fig. 2). Dans ce cas la perfusion peut être continue ou discontinue selon la nécessité de l'expérience. De plus, le pilotage de cette perfusion peut être régulé de façon automatique en fonction de paramètres mesurés par des capteurs appropriés (non représentés) placés au niveau des unités périphériques de culture (19) et asservissant les vannes (24), (25) et pompes (22) utilisées, par l'intermédiaire de circuits électroniques convenablement adaptés et programmés.

Le dispositif périphérique de culture (19) fait appel à des unités dites "chambres de culture thermostatées à perfusion".

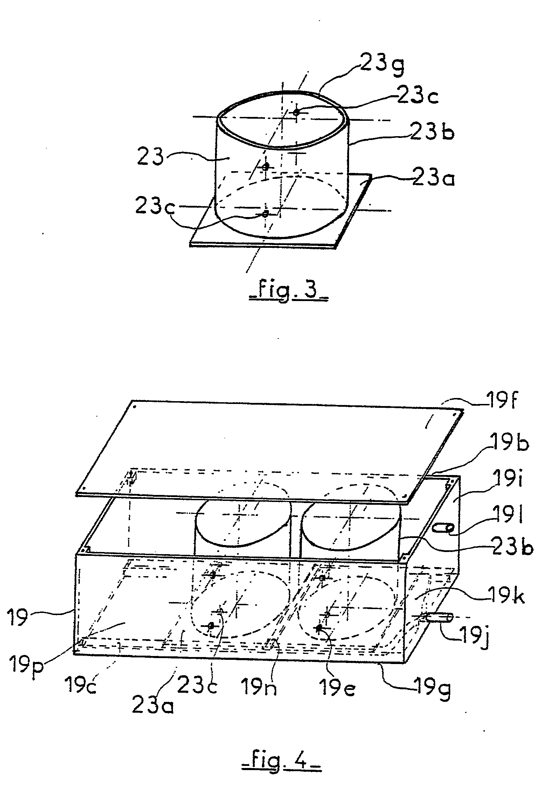

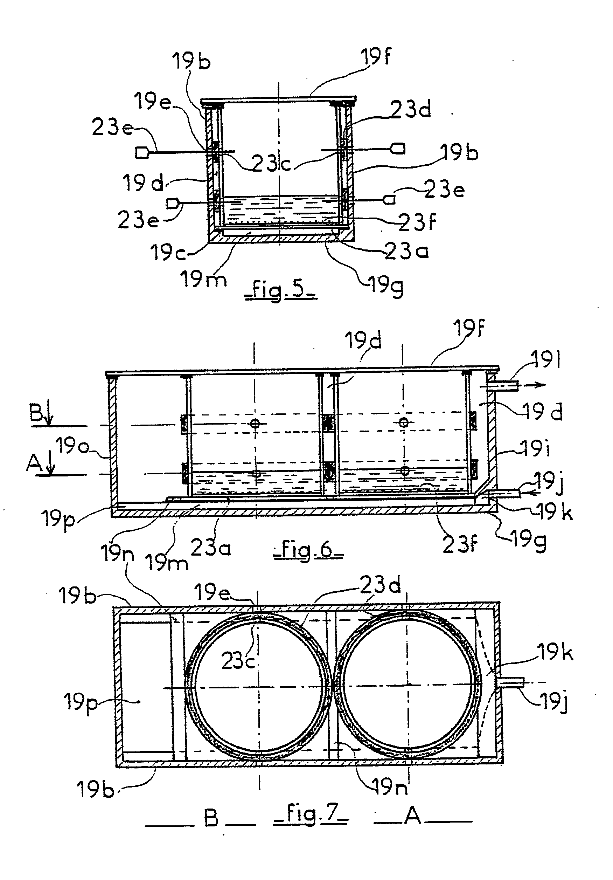

Ces chambres dont un exemple de détail est montré sur les figures 3 à 7 sont constituées principalement de deux parties : le (ou les) "pots de culture perfusables" (23) d'une part et la chambre de thermostation à circulation de liquide caloporteur (19) d'autre part.

Le "pot de culture perfusable" (23) est formé, d'une part d'un fond plat (23a) optiquement correct pour l'observation en transmission et servant de support à la culture en mono-couche ou à la lamelle pré-fragmentée (23f) sur laquelle la culture est conduite dans le cas d'une culture cellulaire et d'un récipient cylindrique ou parallélépipédique (23b) soudé à ce fond ou moulé d'une pièce avec lui. Ce récipient comporte des orifices diamétralement opposés par paire (23c), deux en cas d'oxygénation du milieu de culture dans l'unité centrale, quatre en cas d'oxygénation au niveau du pot de culture, deux en position supérieure en cas de cultures anaérobies.

Ces orifices sont obstrués par paire par des joints bagues collés (23d) en silicone pur ou tout autre matériau approprié et permettant au travers des orifices (23c) le cathétérisme (23e) nécessaire aux perfusions de milieu et (ou) de gaz.

Les cotes de ces pots de culture sont fonction de la dimension et des cotes des chambres de thermostation décrites plus bas ; en particulier le fond (23a) doit pouvoir s'insérer entre les parois latérales (19b) de cette chambre et venir reposer sur les épaulements (19c) de son fond entre les entretoises (19n).

Par ailleurs le diamètre de la partie cylindrique (23b) est établi de telle sorte que puisse s'établir entre sa paroi externe et les parois internes de la chambre de thermostation une chemise de liquide de thermostation (19d) suffisante.

Le ou les joints annulaires (23d) circonscrivant le pot au niveau des paires d'orifices de perfusion (23c) sont réalisés en matière souple, élastique et atoxique comme du silicone pur, et ont une épaisseur telle qu'ils soient insérables aisément entre les parois latérales de la chambre de thermostation (19b) et qu'alors comprimés entre les parois verticales du pot (23b) et celles de la chambre (19b) une étanchéité soit assurée au niveau des orifices de perfusion du pot de culture (23c) et de la chambre thermostatée (19e) tout en permettant le cathétérisme (23e) et la perfusion de ce pot au travers dudit joint annulaire (23d) à l'aide d'aiguilles de type hypodermique.

Le pot de culture peut être réalisé en verre mais également en toute autre matière dont l'état de surface et l'absence de toxicité sont compatibles avec la culture choisie et dont les qualités optiques sont celles requises pour son observation, en particulier en microscopie optique à contraste de phase.

Le bord supérieur du pot (23b) reçoit un profilage tel qu'il s'adapte parfaitement au joint prévu à cet effet sur le couvercle (19f) de la chambre de thermostation notamment joint torique, hémi-torique, plan ou à simple ou double lèvre, ou toute autre combinaison couvercle de "chambre-pot" assurant une rigoureuse herméticité.

La chambre de thermostation de forme cubique ou parallélépipédique en général, mais éventuellement de toute autre forme compatible avec celle du ou des pots de culture utilisés est caractérisée principalement par un fond optiquement plan et transparent (19g), par des parois latérales (19b) comportant des orifices (19e) placés de chaque côté en opposition deux par deux, à des niveaux propres à être en regard de ceux du pot de culture correctement inséré.

Une des parois frontales (19i) comporte dans sa partie la plus basse et en son milieu, l'orifice d'admission (19j) de l'eau ou de tout autre fluide caloporteur destiné à la thermostation, en correspondance avec le diffuseur (19k). La même paroi comporte dans sa partie la plus haute l'orifice de sortie pour retour du fluide caloporteur (19l).

A la jonction des parois latérales (19b) et du fond (19g) sont situées deux réglettes d'épaulement (19c) destinées à recevoir les pots de culture maintenant entre le fond de ceux-ci (23a) et le fond de la chambre de thermostation (19g) un espace creux (19m) destiné à être parcouru par une lame de fluide caloporteur transparent circulant à ce niveau en régime laminaire depuis le diffuseur (19k) assurant ainsi le maintien à la température convenable de la surface de culture formée par le fond du pot (23a) et éventuellement la lamelle pré-fragmentée (23f). Ces deux épaulements sont éventuellement reliés entre eux, à leur face supérieure, en particulier pour les chambres parallélépipédiques destinées à recevoir plusieurs pots de culture, par des entretoises (19n) destinées à positionner les différents pots de culture.

Le diffuseur (19k) formant également, par sa partie supérieure, entretoise frontale, assure la diffusion du fluide caloporteur en une lame horizontale thermostatante.

La dernière entretoise, placée en opposition au diffuseur et positionnant dans la chambre le dernier pot de culture est placée à une distance correcte de la paroi frontale la plus proche (19o) ménageant ainsi un orifice (19p) permettant à la lame d'eau ou de fluide caloporteur de remonter dans la chambre et de constituer autour des parois des pots des chemises humides assurant la thermostation de leur contenu liquide et gazeux ; le liquide caloporteur, après avoir constitué ces chemises calovectrices s'échappe par l'orifice supérieur (19l) de la paroi frontale dite d'admission (19i) avant de retourner à l'unité centrale.

L'ensemble de la chambre de thermostation et du ou des pots de culture est coiffé d'un couvercle (19f) optiquement plan et transparent assurant la fermeture supérieure étanche des deux éléments. Il comporte pour ce faire un joint périphérique l'adaptant intimement aux parois verticales de la chambre et des joints correctement positionnés pour assurer la fermeture supérieure du ou des pots selon les dispositions précédemment énumérées. Il comporte de plus, et en rapport avec les parois verticales de la chambre de thermostation, les dispositifs propres à sa fermeture et à son maintien en position lors de l'utilisation de l'unité périphérique.

L'ensemble fermé de la chambre de thermostation, du ou des pots de culture et du couvercle forme donc une double enceinte caractérisée par un ou des volumes perfusables centraux, destinés à la culture et une enceinte périphérique où circule l'eau ou tout autre fluide caloporteur, afin de maintenir, quelles que soient les circonstances, une température adéquate dans les volumes centraux ; le support de culture est thermostaté par la lame fluide directement de l'unité centrale tandis que le milieu nutritif et (ou) les gaz nécessaires aux métabolismes sont maintenus en température par les chemises humides assurant autour des pots le retour du fluide caloporteur vers l'unité centrale.

L'ensemble de ce dispositif ainsi fermé permet également la réalisation de culture dans des circonstances particulières de micro-gravité. L'enceinte périphérique autour du ou des pots de culture forme un espace étanche et la circulation du fluide caloporteur s'effectue dans un circuit fermé et pressurisé.

L'ensemble de ce dispositif est également caractérisé par ses propriétés optiques, permettant, au travers du couvercle, du milieu de culture, du fond du pot, de la lame d'eau ou de fluide caloporteur et du fond de la chambre de thermostation une observation, notamment optique, du processus de culture.

Bien entendu l'invention n'est pas limitée aux exemples d'exécution ci-dessus décrits et représentés pour lesquels on pourra prévoir d'autres variantes sans pour cela sortir du cadre des revendications annexées, ainsi que par exemple, la nature et la forme des pots de culture qui peut varier dans de larges proportions, de même que leur organisation interne liée à la sorte d'utilisation que l'on en désire faire.

## Revendications

1. Procédé de culture "in vitro", en particulier de cellules homéothermes, du type consistant à établir une culture dans une enceinte dite "pot de culture" (23) comportant un milieu nutritif et munie de moyens de thermostation, en vue de l'observation de l'évolution de la culture, caractérisé en ce qu'il consiste à établir, au moins sous le pot (23) une circulation de fluide caloporteur maintenu à une température adaptée à l'environnement et à l'expérimentation, ledit fluide caloporteur étant transparent tout comme les parois horizontales du pot (23), pour permettre l'observation continue de l'évolution de la culture, en dehors de toute étuve et notamment l'automatisation du pilotage en fonction de paramètres optiques.

2. Procédé de culture "in vitro" selon la revendication 1, caractérisé en ce qu'il consiste:
   – à placer le pot de culture (23) dans une enceinte thermostatée (19),
   – à établir une circulation de fluides nutritifs nécessaires à la culture dans la double enceinte ainsi formée,
   – à établir au moins sous et autour du pot (23), à l'intérieur de la chambre de thermostation (19), une circulation de fluide caloporteur maintenu à une température adaptée à l'environnement et à l'expérimentation, ledit fluide caloporteur étant transparent tout comme les parois horizontales (19f, 23a, 23f, 19g) de la chambre (19) et du pot (23), pour permettre l'obsevation continue de l'évolution de la culture.

3. Procédé de culture "in vitro" selon l'une quelconque des revendications 1 ou 2, caractérisé en ce qu'il consiste à établir une circulation de fluide caloporteur pressurisé dans la chambre de thermostation (19) permettant, en particulier, le développement et l'observation de la culture dans des conditions de micro-gravité.

4. Installation pour la mise en œuvre du procédé selon l'une quelconque des revendications 1 à 3, caractérisée en ce qu'elle est constituée d'au moins un pot de culture (23) sus le fond (23a) duquel circule une lame de fluide caloporteur transparent provenant de et retournant à une unité centrale de thermostation, laquelle lame parcourt un espace creux (19m) prévu entre le fond (23a) du pot (23) et le fond (19g) d'une chambre de thermostation (19), au moins les faces horizontales de la chambre de thermostation (19) et du pot de culture (23) étant réalisées en matière transparente, de manière à permettre l'observation, en particulier optique, de la culture.

5. Installation selon la revendication 4, caractérisée en ce qu'elle est constituée:
   – d'au moins un pot de culture (23) muni de moyens d'alimentation en fluides nutritifs,
   – d'une chambre thermostatée (19) définissant de toutes parts, sauf éventuellement à la surface supérieure dudit pot (23), un espace destiné à la circulation d'un fluide caloporteur provenant de et retournant à une unité centrale de thermostation, au moins les faces horizontales de la chambre thermostatée (19) où circule le fluide caloporteur transparent: couvercle (19f) et le socle (19g) et le fond (23a) du pot (23) étant réalisées en matière transparente de manière à permettre l'observation, en particulier optique, de la culture et, selon le cas, l'automatisation du pilotage de ladite culture en fonction de paramètres optiques.

6. Installation selon la revendication 5, caractérisée en ce que le fond (19g) de la chambre (19) est muni de deux épaulements (19c) destinés à recevoir les pots de culture (23) et à maintenir entre leur fond (23a) et le fond (19g) de ladite chambre de thermostation (19), un espace creux (19m) destiné à être parcouru par la lame de fluide caloporteur.

7. Installation selon la revendication 5 ou 6 caractérisée en ce que le pot de culture (23) et la chambre de thermostation (19) sont pourvus d'orifices apairés (23c et 19e) susceptibles d'être mis en communication avec les réservoirs de fluide nécessaire au déroulement de la culture, notamment à l'aide d'un cathéter, un joint étant interposé à chaque fois entre la paroi externe du pot (23b) et la paroi interne de la chambre (19), ce joint étant réalisé en toute matière élastique propre à assurer d'une part l'étanchéité entre pot et chambre thermostatée et, d'autre part, entre chambre thermostatée et l'extérieur.

8. Installation selon l'une quelconque des revendications 5 à 7 caractérisée en ce que la chambre de thermostation (19) comporte un couvercle transparent (19f) amovible assurant la fermeture étanche du pot après son inoculation et la fermeture, également étanche, de ladite chambre de thermostation (19) permettant ainsi la circulation continue autour du pot (23), du fluide caloporteur thermostatant.

9. Installation selon la revendication 8, caractérisée en ce que la circulation du fluide caloporteur autour du ou des pots s'effectue au moyen d'un circuit fermé et pressurisé.

10. Installation selon l'une quelconque des revendications 5 à 9 caractérisée en ce que la circulation du milieu de culture entre un réservoir oxygénateur et le pot de culture est assurée, pour certaines applications, telle que la perfusion de la culture en circuit ouvert, à partir d'une réserve de gaz sous pression servant à la fois à l'oxygénation du milieu et à sa propulsion au travers du pot de culture en fonction des nécessités de celle-ci.

11. Installation selon l'une quelconque des revendications 5 à 10, adaptée à la culture des flores microbiennes anaérobies soit sous vide, soit sous atmosphère gazeuse appropriée, en milieu liquide ou sur gel.

12. Installation selon l'une quelconque des revendications 5 à 11, caractérisée en ce que le pot de culture (23) peut être aménagé de façon à permettre des tests standard de biomatériaux mis en présence des cultures, tests par tout dispositif approprié (logettes, films d'Agar, ou de collagène, filtres de type Milipore, etc...)

## Claims

1. "In vitro" culture process, especially for homoiothermic cells, of the type consisting in establishing a culture in a chamber referred to as a "culture pot" (23) containing a nutrient medium and provided with thermostating means, for the purpose of observation of the development of the culture, characterized in that it consists in establishing, at least under the pot (23), a circulation of heat-transfer fluid maintained at a temperature suited to the environment and to the experiment, the said heat-transfer fluid being transparent, like the horizontal walls of the pot (23), to allow continuous observation of the development of the culture outside any incubator and, in particular, automation of control in accordance with optical parameters.

2. "In vitro" culture process according to Claim 1, characterized in that it consists in:
   - placing the culture pot (23) in a thermostated chamber (19),
   - establishing circulation of nutrient fluids necessary to the culture in the double chamber thus formed,
   - establishing at least under and around the pot (23), inside the thermostating chamber (19), circulation of heat-transfer fluid maintained at a temperature suited to the environment and to the experiment, the said heat-transfer fluid being transparent, like the horizontal walls (19f, 23a, 23f, 19g) of the chamber (19) and of the pot (23), to allow continuous observation of the development of the culture.

3. "In vitro" culture process according to either of Claims 1 and 2, characterized in that it consists in establishing circulation of pressurized heat-transfer fluid through the thermostating chamber (19) allowing, in particular, the development and observation of the culture under microgravity conditions.

4. Installation for carrying out the process according to any one of Claims 1 to 3, characterized in that it consists of at least one culture pot (23), under the bottom (23a) from which circulates a layer of transparent heat-transfer fluid coming from and returning to a central thermostating unit, which layer passes through a hollow space (19m) provided between the bottom (23a) of the pot (23) and the bottom (19g) of a thermostating chamber (19), at least the horizontal faces of the thermostating chamber (19) and of the culture pot (23) being made of transparent material so as to permit observation, especially optical observation, of the culture.

5. Installation according to Claim 4, characterized in that it consists of:
   - at least one culture pot (23) provided with means of supply of nutrient fluids,
   - a thermostated chamber (19) defining on all sides, except possibly at the upper surface of the said pot (23), a space intended for circulation of a heat-transfer fluid coming from and returning to a central thermostating unit, at least the horizontal faces of the thermostated chamber (19) through which the transparent heat-transfer fluid circulates, namely the lid (19f), the base (19g) and the bottom (23a) of the pot (23) being made of transparent material so as permit observation, especially optical observation, of the culture and, depending on the case, automation of the control of the said culture in accordance with optical parameters.

6. Installation according to Claim 5, characterized in that the bottom (19g) of the chamber (19) is provided with two shoulders (19c) made to receive the culture pots (23) and to maintain between their bottom (23a) and the bottom (19g) of the said thermostating chamber (19) a hollow space (19m) made to be crossed by the layer of heat-transfer fluid.

7. Installation according to Claim 5 or 6, characterized in that the culture pot (23) and the thermostating chamber (19) are provided with matched

orifices (23c and 19e) capable of being brought into communication with the reservoirs of fluid necessary for the development of the culture, in particular by means of a catheter, a gasket being interposed in each instance between the outer wall of the pot (23b) and the inner wall of the chamber (19), this gasket being made of any elastic material suitable for providing the tightness, on the one hand between the pot and the thermostated chamber, and on the other hand between the thermostated chamber and the outside.

8. Installation according to any one of Claims 5 to 7, characterized in that the thermostating chamber (19) comprises a removable transparent lid (19f) providing for leakproof closing of the pot after its inoculation and closing, also leakproof, of the said thermostating chamber (19), thereby allowing the continuous circulation of the thermostating heat-transfer fluid aroud the pot (23).

9. Installation according to Claim 8, characterized in that the circulation of the heat-transfer fluid around the pot or pots is made by means of a closed and pressurized circuit.

10. Installation according to any one of Claims 5 to 9, characterized in that the circulation of the culture medium between an oxygenation reservoir and the culture pot is made for some applications such as open-circuit perfusion of the culture, from a reserve of gas under pressure serving both for oxygenation of the medium and its propulsion through the culture pot in accordance with the requirements of the culture.

11. Installation according to any one of Claims 5 to 10, suited to the culture of anaerobic microbial flora either under vacuum or under a suitable gaseous atmosphere, in a liquid medium or on gel.

12. Installation according to any one of Claims 5 to 11, characterized in that the culture pot (23) may be realized so as to allow standard tests on biomaterials brought into contact with the cultures, tests by any suitable device (housings, agar or collagen films, Millipore type filters, and the like).

**Patentansprüche**

1. "In vitro"-Kultivierungsverfahren, insbesondere von homöothermen Zellen, das darin besteht, eine Kultur in einem Behälter, der als "Kulturgefäß" (23) bezeichnet wird und ein Nährmedium enthält, sowie mit einer Thermostatisierungseinrichtung versehen ist, aufzuziehen, wobei die Entwicklung der Kultur beobachtet wird, dadurch gekennzeichnet, daß es daraus besteht, wenigstens unter dem Gefäß (23) einen Kreislauf von Wärmeleitender Flüssigkeit einzurichten, der auf einer der Umgebung und dem Experimentieren angepaßten Temperatur gehalten wird, wobei die wärmeleitende Flüssigkeit sowie die horizontalen Wände des Gefäßes (23) transparent sind, wobei die kontinuierliche Beobachtung der Entwicklung der Kultur ohne jegliche Stabilisierungseinrichtung und insbesondere die Automatisation der Steuerung in Abhängigkeit von optischen Parametern zu ermöglichen.

2. "In vitro"-Kultivierungsverfahren nach Anspruch 1, dadurch gekennzeichnet, daß es darin besteht, das Kulturgefäß (23) in einen thermostatisierten Behälter (19) zu stellen, einen Kreislauf der für die Kultur notwendigen Nährlösungen in dem so geformten Doppelgefäß zu schaffen und wenigstens unter und um das Topfgefäß (23) im Inneren der Thermostatkammer (19) einen Kreislauf der wärmeleitenden Flüssigkeit, die auf einer der Umgebung und dem Experimentieren angepaßten Temperatur gehalten wird, zu schaffen, wobei die wärmeleitende Flüssigkeit sowie die horizontalen Wände (19f), (23a), (23f), (19g) der Kammer (19) und des Gefäßes (23) transparent sind, um so die kontinuierliche Beobachtung der Entwicklung der Kultur zu ermöglichen.

3. "In-vitro"-Kultivierungsverfahren nach irgendeinem der Ansprüche 1 oder 2, dadurch gekennzeichnet, daß es darin besteht, einen Kreislauf einer wärmeleitenden Flüssigkeit zu schaffen, die in der Thermostatkammer (19) unter Druck gesetzt ist, um insbesondere die Entwicklung und die Beobachtung der Kultur unter den Bedingungen der Mikrogravität zu erlauben.

4. Vorrichtung zur Durchführung des Verfahrens nach irgendeinem der Ansprüche 1 bis 3, dadurch gekennzeichnet, daß sie wenigstens ein Kulturgefäß (23) aufweist, unter dessen Boden (23a) eine wärmeleitende, transparente Flüssigkeit zirkuliert, die von einer zentralen Thermostatisierungsvorrichtung ausgeht und zu dieser zurückfließt, wobei diese Flüssigkeit einen Hohlraum (19m) durchläuft, der zwischen dem Boden (23a) des Gefäßes (23) und dem Boden (19g) einer Thermostatkammer (19) vorgesehen ist, wobei wenigstens die horizontalen Seiten der Thermostatkammer (19) und des Kulturgefäßes (23) aus einem transparenten Material bestehen, das insbesondere die optische Beobachtung der Kultur ermöglicht.

5. Vorrichtung nach Anspruch 4, dadurch gekennzeichnet, daß sie wenigstens ein Kulturgefäß (23) aufweist, das mit Mitteln für die Versorgung mit Nährlösungen ausgerüstet ist sowie eine Thermostatkammer (19) aufweist, die von allen Seiten, außer gegebenenfalls auf der Oberfläche des Gefäßes (23) einen Raum bildet, der für den Kreislauf einer wärmeleitenden Flüssigkeit bestimmt ist, die von einer zentralen Thermostatisierungseinheit ausgeht und zu dieser zurückläuft und wobei wenigstens die horizontalen Seiten der Thermostatkammer (19), in der die wärmeleitende transparente Flüssigkeit zirkuliert, d.h. der Deckel (19f), der Sockel (19g) und der Boden (23a) des Gefäßes (23) aus einem transparenten Material bestehen, das insbesondere die optische Beobachtung der Kultur erlaubt und gegebenenfalls die Automatisation der Steuerung der genannten Kultur in Abhängigkeit von optischen Parametern erlaubt.

6. Vorrichtung nach Anspruch 5, dadurch gekennzeichnet, daß der Boden (19g) der Kammer (19) mit zwei Vorsprüngen (19c) versehen ist, die dafür bestimmt sind, die Kulturgefäße (23) aufzunehmen und zwischen ihrem Boden (23a) und dem Boden (19g) der Thermostatkammer (19) einen Hohlraum (19m) aufrechtzuerhalten, der dazu bestimmt ist, von der wärmeleitenden Flüssigkeit durchflossen zu werden.

7. Vorrichtung nach einem der Ansprüche 5 oder 6, dadurch gekennzeichnet, daß in dem Kulturgefäß (23) und der Thermostatkammer (19) paarweise Löcher (23c) und (19e) vorgesehen sind, die geeignet sind, eine Verbindung mit den Flüssigkeitsreservoirs, die für die Entwicklung der Kultur notwendig sind, herzustellen, insbesondere mit Hilfe eines Katheters, wobei eine Dichtung jedesmal zwischen der äußeren Wand des Topfes (23b) und der inneren Wand der Kammer (19) angebracht ist, wobei diese Dichtung aus einem elastischen Material gefertigt ist, das einerseits dazu geeignet ist, die Dichtigkeit zwischen Gefäß und Thermostatkammer und andererseits zwischen Thermostatkammer und äußerer Umgebung sicherzustellen.

8. Vorrichtung nach irgendeinem der Ansprüche 5 bis 7, dadurch gekennzeichnet, daß die Thermostatkammer (19) einen transparenten Deckel (19f) aufweist, der entfernbar ist und der einen dichten Verschluß des Gefäßes nach seiner Beimpfung sicherstellt, wobei auf diese Weise der ebenfalls dichte Verschluß der Thermostatkammer (19) in der Weise den fortgesetzten Fluß der thermostatisierten, wärmeleitenden Flüssigkeit um das Gefäß (23) herum erlaubt.

9. Vorrichtung nach Anspruch 8, dadurch gekennzeichnet, daß der Kreislauf der wärmeleitenden Flüssigkeit um das/die Gefäß/Gefäße mittels eines geschlossenen und unter Druck stehenden Kreislaufs durchgeführt wird.

10. Vorrichtung nach irgendeinem der Ansprüche 5 bis 9, dadurch gekennzeichnet, daß der Kreislauf des Nährmediums zwischen einem sauerstoffhaltigen Reservoir und dem Kulturgefäß für bestimmte Anwendungen so durchgeführt wird, daß die Versorgung der Kultur im offenen Kreislauf, ausgehend von einem unter Druck befindlichen Gasvorrat, der gleichzeitig zur Anreicherung des Milieus mit Sauerstoff und der Ausbreitung der Nährlösung durch das Kulturgefäß, je nach Notwendigkeit, dient, sichergestellt ist.

11. Vorrichtung nach irgendeinem der Ansprüche 5 bis 10, die an Kulturen von Floren anaerober Mikroben in Flüssigkeitskultur oder auf Gel, entweder unter Vakuum oder unter einer geeigneten Gasatmosphäre, angepaßt ist.

12. Vorrichtung nach irgendeinem der Ansprüche 5 bis 11, dadurch gekennzeichnet, daß das Kulturgefäß (23) in der Weise ausgestattet sein kann, die es erlaubt, Standardtests von Biomaterialien sowie Tests für geeignete Vorrichtungen in Gegenwart der Kulturen durchzuführen (Logettes, Streifen von Agar oder Collagen, Miliporfilter, etc.).

_fig.1_

EP 0 222 644 B1

fig.2

_fig. 3_

_fig. 4_

_fig.5_

_fig.6_

_fig.7_